# FASCICULE DE BREVET EUROPEEN

(11) **EP 0 663 908 B1**
(45) Date de publication et mention de la délivrance du brevet: **12.02.1997**
(21) Numéro de dépôt: 93921984.6
(22) Date de dépôt: 04.10.1993
(51) Int. Cl.: C07D 305/14, C07D 263/04, C07D 413/12

(54) **PROCEDE DE PREPARATION DE DERIVES DU TAXANE**
VERFAHREN ZUR HERSTELLUNG VON TAXAN-DERIVATEN
METHOD OF PREPARING TAXANE DERIVATIVES

(30) Priorité: 05.10.1992 FR 9211743
(43) Date de publication de la demande: 26.07.1995
(73) Titulaire: RHONE-POULENC RORER S.A., 92160 Antony (FR)
(72) Inventeur: COMMERCON, Alain, F-94400 Vitry sur Seine (FR); DIDIER, Eric, F-75013 Paris (FR); FOUQUE, Elie, F-94100 Saint Maur des Fosses (FR)
(74) Mandataire: Pilard, Jacques
(86) Numéro de dépôt international: FR9300969
(87) Numéro de publication internationale: WO9407879

(56) Documents cités:
- WO-A-92/09589
- J. Med. Chem. 1991, vol. 34, pages 994-998

## Description

La présente invention concerne un nouveau procédé de préparation de dérivés du taxane de formule générale : 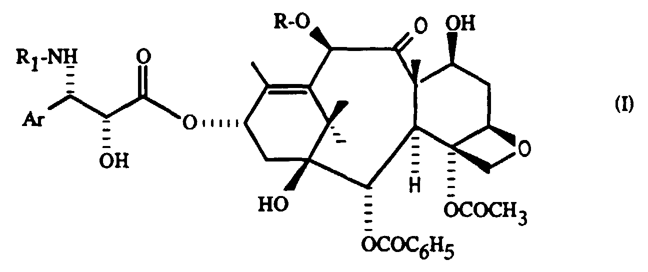 qui présentent des propriétés antileucémiques et antitumorales remarquables.

Dans la formule générale (I):
R représente un atome d'hydrogène ou un radical acétyle, R₁ représente un radical benzoyle ou un radical R₂-O-CO- dans lequel R₂ représente un radical alcoyle, alcényle, alcynyle, cycloalcoyle, cycloalcényle, bicycloalcoyle, phényle ou hétérocyclyle azoté, et Ar représente un radical aryle.

Plus particulièrement, R représente un atome d'hydrogène ou un radical acétyle et R₁ représente un radical benzoyle ou un radical R₂-O-CO- dans lequel R₂ représente:
- un radical alcoyle droit ou ramifié contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone ou bicycloalcoyle contenant 7 à 10 atomes de carbone, ces radicaux étant éventuellement substitués par un ou plusieurs substituants choisis parmi les atomes d'halogène et les radicaux hydroxy, alcoyloxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle, cyano, carboxy ou alcoyloxycarbonyle dont la partie alcoyle contient 1 à 4 atomes de carbone,
- ou un radical phényle éventuellement substitue par un ou plusieurs atomes ou radicaux choisis parmi les radicaux alcoyles contenant 1 à 4 atomes de carbone ou alcoyloxy contenant 1 à 4 atomes de carbone,
- ou un radical hétérocyclyle azoté saturé ou non saturé contenant 5 ou 6 chaînons éventuellement substitué par un ou plusieurs radicaux alcoyles contenant 1 à 4 atomes de carbone,
étant entendu que les radicaux cycloalcoyles, cycloalcényles ou bicycloalcoyles peuvent être éventuellement substitués par un ou plusieurs radicaux alcoyles contenant 1 à 4 atomes de carbone, et
Ar représente un radical phényle ou α- ou β-naphtyle éventuellement substitué par un ou plusieurs atomes ou radicaux choisis parmi les atomes d'halogène (fluor, chlore, brome, iode) et les radicaux alcoyles, alcényles, alcynyles, aryles, arylalcoyles, alcoxy, alcoylthio, aryloxy, arylthio, hydroxy, hydroxyalcoyle, mercapto, formyle, acyle, acylamino, aroylamino, alcoxycarbonylamino, amino, alkylamino, dialkylamino, carboxy, alcoxycarbonyle, carbamoyle, dialcoylcarbamoyle, cyano et trifluorométhyle, étant entendu que les radicaux alcoyles et les portions alcoyles des autres radicaux contiennent 1 à 4 atomes de carbone, que les radicaux alcényles et alcynyles contiennent 3 à 8 atomes de carbone et les radicaux aryles sont les radicaux phényles ou α- ou β-naphtyles.

D'un intérêt tout particulier sont les produits de formule générale (I) dans laquelle R représente un atome d'hydrogène ou un radical acétyle, R₁ représente un radical benzoyle ou t.butoxycarbonylamino et Ar représente un radical phényle.

Les produits de formule générale (I) dans laquelle R₁ représente un radical benzoyle correspondent au taxol et au désacétyl-10 taxol et les produits de formule générale (I) dans laquelle R₁ représente un radical t.butoxycarbonyle correspondent à ceux qui font l'objet du brevet européen EP 0 253 738.

Dans J. Med. Chem., 34, 992 (1991) est décrite la préparation de produits de formule générale (I) dans laquelle R₁ représente un atome d'hydrogène, qui sont inactifs, par une réaction d'oxyamination de Sharpless.

Selon le procédé qui est décrit dans la demande internationale PCT WO 92/09589, les dérivés de formule générale (I) peuvent être obtenus par :
- condensation d'un dérivé de l'oxazolidine de formule générale: 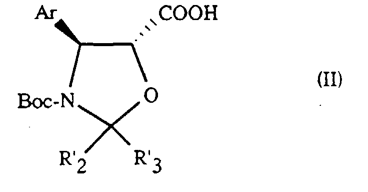 dans laquelle Ar est défini comme précédemment, Boc représente le radical t.butoxycarbonyle et R'₂ et R'₃, identiques ou différents, représentent un radical alcoyle contenant 1 à 4 atomes de carbone éventuellement substitué par un ou plusieurs radicaux aryles, ou un radical aryle, ou bien R'₂ et R'₃ forment ensemble avec l'atome de carbone auquel ils sont liés un cycle ayant de 4 à 7 chaînons, sur la baccatine III ou la désacétyl-10 baccatine III protégée de formule générale: 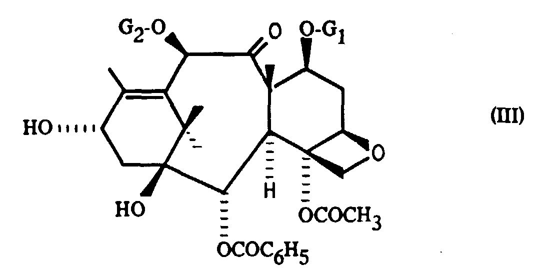 dans laquelle G₁ représente un groupement protecteur de la fonction hydroxy et G₂ représente un radical acétyle ou un groupement protecteur de la fonction hydroxy, pour obtenir un produit de formule générale : 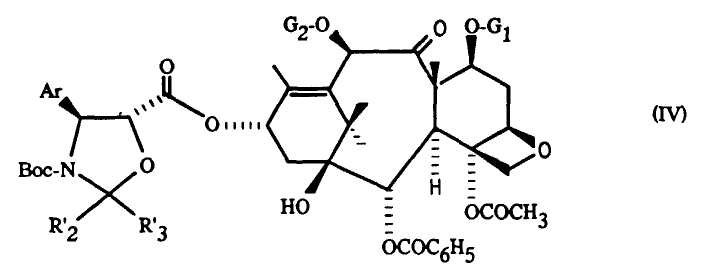 dans laquelle Ar, R'₂, R'₃, G₁, G₂ et Boc sont définis comme précédemment,
- traitement en milieu acide du produit de formule générale (IV) dans des conditions qui sont sans effet sur G₁ et G₂ pour obtenir le produit de formule générale : 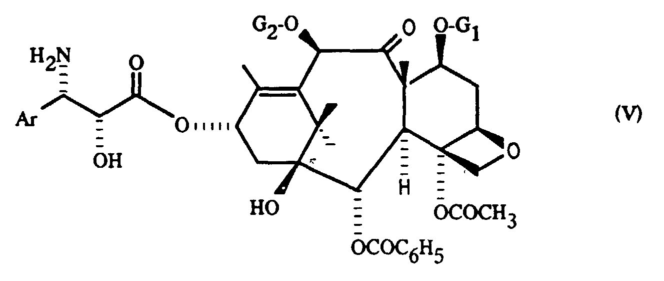 dans laquelle Ar, G₁ et G₂ sont définis comme précédemment,
- traitement du produit de formule générale (V) par un réactif convenable pour introduire un radical R₁, c'est-à-dim un radical benzoyle ou R₂-O-CO-, pour obtenir un produit de formule générale: 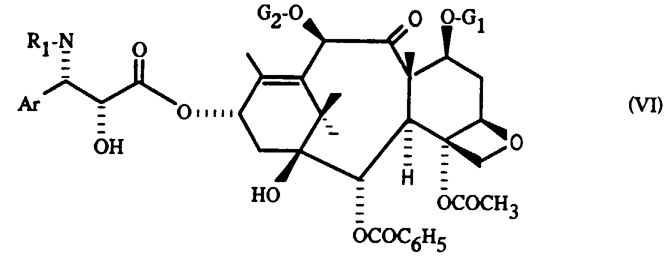 dans laquelle Ar, R₁, G₁ et G₂ sont définis comme précédemment, et
- remplacement des groupements protecteurs G₁ et G₂ du produit de formule générale (VI) par des atomes d'hydrogène pour obtenir le produit de formule générale (I).

Il a maintenant été trouvé, et c'est ce qui fait l'objet de la présente invention, que les produits de formule générale (I) peuvent être obtenus :
1) en estérifiant la baccatine III ou la désacétyl-10 baccatine III protégée de formule générale (III) dans laquelle G₁ et éventuellement G₂ représentent un groupement protecteur de la fonction hydroxy au moyen d'un acide de formule générale : 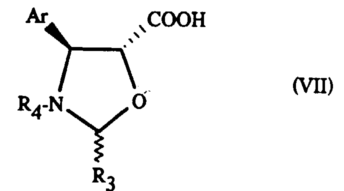 dans laquelle Ar est défini comme précédemment, R₃ représente un radical trihalométhyle, de préférence trichlorométhyle ou phényle substitué par un radical trihalométhyle, de préférence trichlorométhyle, ou d'un dérivé de cet acide, et R₄ représente un atome d'hydrogène ou est identique à R₁ défini comme précédemment, pour obtenir un produit de formule générale : 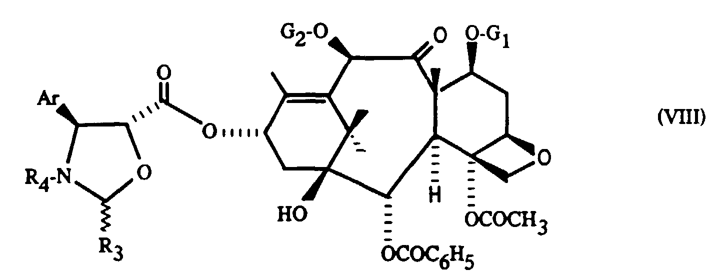 dans laquelle Ar, R₃, R₄, G₁ et G₂ sont définis comme précédemment,
2) en remplaçant des groupements protecteurs des fonctions hydroxy et amino du produit de formule générale (VIII) par des atomes d'hydrogène pour obtenir le produit de formule : 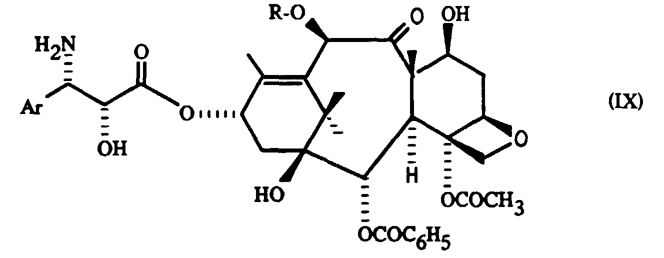
3) en traitant le produit obtenu de formule générale (IX) par un réactif qui permet d'introduire un substituant R₁ sur la fonction amino pour obtenir un produit de formule générale (I).

Selon la présente invention, l'estérification de la baccatine III ou de la désacétyl-10 baccatine III protégée de formule générale (III) par un acide de formule générale (VII), dans laquelle R₄ représente de préférence un atome d'hydrogène, peut être effectuée en présence d'un agent de condensation tel qu'un diimide comme le dicyclohexylcarbodiimide ou un carbonate réactif comme le dipyridyl-2 carbonate et d'un agent d'activation tel qu'une aminopyridine comme la diméthylamino-4 pyridine ou la pyrrolidino-4 pyridine en opérant dans un solvant organique choisi parmi les éthers tels que le tétrahydrofuranne, le diisopropyléther, le méthyl t.butyléther ou le dioxanne, les cétones telles que la méthylisobutylcétone, les esters tels que l'acétate d'éthyle, l'acétate d'isopropyle ou l'acétate de n.butyle, les nitriles tels que l'acétonitrile, les hydrocarbures aliphatiques tels que le pentane, l'hexane ou l'heptane, les hydrocarbures aliphatiques halogénés tels que le dichlorométhane ou le dichloro-1,2 éthane et les hydrocarbures aromatiques tels que le benzène, le toluène, les xylènes, l'éthylbenùne, l'isopropylbenzéne ou le chlorobenzène à une température comprise entre -10 et 90°C. Il est particulièrement avantageux d'effectuer l'estérification en opérant dans un hydrocarbure aromatique à une température voisine de 20°C.

L'estérification peut aussi être réalisée en utilisant l'acide de formule générale (VII) sous forme d'anhydride de formule générale : 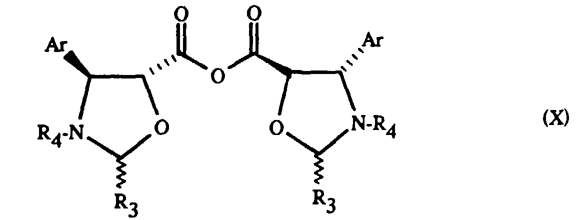 dans laquelle Ar, R₃ et R₄ sont définis comme précédemment, en présence d'un agent d'activation tel qu'une aminopyridine comme la diméthylamino-4 pyridine ou la pyrrolidino-4 pyridine en opérant dans un solvant organique choisi parmi les éthers tels que le tétrahydrofuranne, le diisopropyléther, le méthyl t.butyléther ou le dioxanne, les cétones telles que la méthylisobutylcétone, les esters tels que l'acétate d'éthyle, l'acétate d'isopropyle ou l'acétate de n.butyle, les nitriles tels que l'acétonitrile, les hydrocarbures aliphatiques tels que le pentane, l'hexane ou l'heptane, les hydrocarbures aliphatiques halogénés tels que le dichlorométhane ou le dichloro1,2 éthane et les hydrocarbures aromatiques tels que le benzène, le toluène, les xylènes, l'éthylbenzène, l'isopropylbenzène ou le chlorobenzène à une température comprise entre 0 et 90°C.

L'estérification peut aussi être réalisée en utilisant l'acide de formule générale (VII) sous forme d'halogénure ou d'anhydride mixte de formule générale : 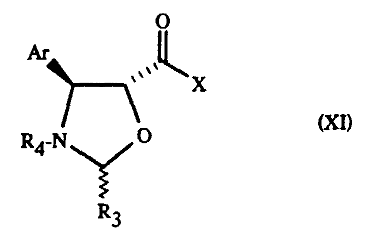 dans laquelle Ar, R₃ et R₄ sont définis comme précédemment, R₄ représentant de préférence un atome d'hydrogène, et X représente un atome d'halogène ou un radical acyloxy ou aroyloxy, éventuellement préparé in situ, en présence d'une base qui est de préférence une base organique azotée telle qu'une amine aliphatique tertiaire, une pyridine ou une aminopyridine comme la diméthylamino-4 pyridine ou la pyrrolidino-4 pyridine en opérant dans un solvant organique inerte choisi parmi les éthers tels que le tétrahydrofuranne, l'éther diisopropylique, le méthyl t.butyléther ou le dioxanne, les cétones comme la méthyl t.butylcétone, les esters comme l'acétate d'éthyle, l'acétate d'isopropyle ou l'acétate de n.butyle, les nitriles tels que l'acétonitrile, les hydrocarbures aliphatiques tels que le pentane, l'hexane ou l'heptane, les hydrocarbures aliphatiques halogénés tels que le dichlorométhane ou le dichloro-1,2 éthane et les hydrocarbures aromatiques tels que le benzène, le toluène, les xylènes, l'éthylbenzène, l'isopropylbenzène ou le chlorobenzène à une température comprise entre 10 et 80°C, de préférence voisine de 20°C.

De préférence, on utilise un dérivé activé de formule générale (XI) dans laquelle X représente un atome d'halogène ou un radical acyloxy contenant 1 à 5 atomes de carbone ou aroyloxy dans lequel la partie aryle est un radical phényle éventuellement substitué par 1 à 5 atomes ou radicaux, identiques ou différents, choisis parmi les atomes d'halogène (chlore, brome) et les radicaux nitro, méthyle ou méthoxy.

Le remplacement par des atomes d'hydrogène des groupements protecteurs des fonctions hydroxy et amino du produit de formule générale (VIII), dans laquelle, de préférence, G₁ et éventuellement G₂ représentent un radical trichloro-2,2,2 éthoxycarbonyle ou (trichlorométhyl-2 propoxy)-2 carbonyle, est effectué généralement par traitement par le zinc, éventuellement associé à du cuivre, en pence d'acide acétique à une température comprise entre 20 et 60°C ou au moyen d'un acide minéral ou organique, tel que l'acide chlorhydrique ou l'acide acétique en solution dans un alcool aliphatique contenant 1 à 3 atomes de carbone ou dans un ester aliphatique (acétate d'éthyle, acétate d'isopropyle, acétate de n.butyle) en présence de zinc éventuellement associé à du cuivre.

Le remplacement des groupements protecteurs du produit de formule générale (VIII) par des atomes d'hydrogène peut aussi être effectué par réduction électrolytique.

L'introduction d'un substituant R₁ sur la fonction amino du produit de formule générale (IX) est effectuée par action du chlorure de benzoyle ou d'un dérivé réactif de formule générale :

R₂-O-CO-Y (XII)

dans laquelle R₂ est défini comme précédemment et Y représente un atome d'halogène ou un reste -O-R₂ ou -O-CO-R₂ en opérant dans un solvant organique tel qu'un ester aliphatique comme l'acétate d'éthyle ou un alcool comme le méthanol, l'éthanol, l'isopropanol ou le n.butanol ou un hydrocarbure aliphatique halogéné comme le dichlorométhane en présence d'une base minérale ou organique telle que le bicarbonate de sodium. Généralement la réaction est effectuée à une température comprise entre 0 et 50°C, de péférence voisine de 20°C.

L'acide de formule générale (VII) peut être obtenu par saponification en milieu basique de l'ester de formule générale : 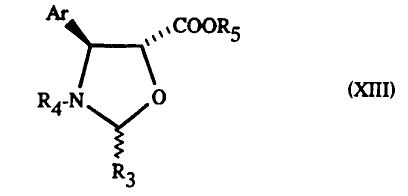 dans laquelle Ar, R₁ et R₄ sont définis comme précédemment et R₅ représente un radical alcoyle contenant 1 à 4 atomes de carbone éventuellement substitué par un radical phényle.

Généralement, la saponification est effectuée au moyen d'une base minérale telle qu'un hydroxyde de métal alcalin (lithium, potassium, sodium), un carbonate ou bicarbonate de métal alcalin (bicarbonate de sodium, carbonate ou bicarbonate de potassium) en milieu hydro-alcoolique tel qu'un mélange méthanol-eau à une température comprise entre 10 et 40°C, de préférence voisine de 20°C.

L'ester de formule générale (XIII) peut être obtenu par action d'un aldéhyde de formule générale :

R₃-CHO (XIV)

dans laquelle R₃ est défini comme précédemment, éventuellement sous forme d'un dialkylacétal, sur un dérivé de la phénylisosérine de formule générale : 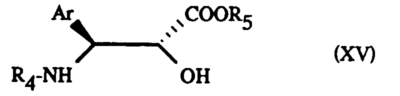 dans laquelle Ar, R₄ et R₅ sont définis comme précédemment, sous forme racémique ou, de préférence sous forme 2R,3S en opérant dans un solvant organique inerte en présence d'un acide fort minéral, tel que l'acide sulfurique, ou organique tel que l'acide p.toluènesulfonique éventuellement sous forme de sel de pyridinium à une température comprise entre 0°C et la température d'ébullition du mélange réactionnel. Les solvants qui conviennent particulièrement bien sont les hydrocarbures aromatiques.

Le produit de formule générale (XV) peut être préparé dans les conditions décrites ou par adaptation des méthodes décrites dans la demande internationale PCT WO 92/09589.

L'anhydride de formule générale (X) peut être obtenu en faisant réagir un agent de déshydratation tel que le dicyclohexylcarbodiimide sur l'acide de formule générale (VII) en opérant dans un solvant organique choisi parmi les éthers tels que le tétrahydrofuranne, le diisopropyléther, le méthyl t.butyléther ou le dioxanne, les cétones telles que la méthylisobutylcétone, les esters tels que l'acétate d'éthyle, l'acétate d'isopropyle ou l'acétate de n.butyle, les nitriles tels que l'acétonitrile, les hydrocarbures aliphatiques tels que le pentane, l'hexane ou l'heptane, les hydrocarbures aliphatiques halogénés tels que le dichlorométhane ou le dichloro-1,2 éthane et les hydrocarbures aromatiques tels que le benzène, le toluène, les xylènes, l'éthylbenzène, l'isopropylbenzène ou le chlorobenzène à une température comprise entre 0 et 30°C.

L'acide activé de formule générale (XI) peut être obtenu par action d'un halogénure de sulfuryle, de préférence le chlorure, ou d'un produit de formule générale:

R₆-CO-Z (XVI)

dans laquelle R₆ représente un radical alcoyle contenant 1 à 4 atomes de carbone ou un radical phényle éventuellement substitué par 1 à 5 atomes ou radicaux, identiques ou différents, choisis parmi les atomes d'halogène et les radicaux nitro, méthyle ou méthoxy et Z représente un atome d'halogène, de préférence un atome de chlore, sur un acide de formule générale (VII) en opérant dans un solvant organique convenable tel que le tétrahydrofuranne en présence d'une base organique telle qu'une amine tertiaire comme la triéthylamine à une température comprise entre 0 et 30°C.

L'exemple suivant illustre la présente invention.

### EXEMPLE

A une solution de 0,33 g d'acide phényl-4 trichlorométhyl-2 oxazolidine-1,3 carboxylique-5-(4S,5R), de 0,49 g d'acétoxy-4 bwaoyloxy-2α époxy-5β,20 dihydroxy-1,13α oxo-9 bis-(trichloroe-2,2,2 éthoxy) carbonyloxy-7β,10β taxène-11 et de 0,013 g de diméthylamino-4 pyridine dans 2,77 cm3 de toluène anhydre, on ajoute, à une température voisine de 20°C, 0,21 g de dicyclohexylcarbodiimide. La solution est agitée à 25°C pendant 2-3 heures puis la dicyclohexylurée formée est filtrée sur un verre fritté. Le précipité est rincé avec 20 cm3 d'acétate d'éthyle et la phase organique est lavée successivement avec 20 cm3 d'une solution aqueuse molaire d'acide chlorhydrique, 20 cm3 d'une solution aqueuse saturée de bicarbonate de sodium et 10 cm3 d'une solution aqueuse saturée de chlorure de sodium. La phase organique est séchée sur sulfate de sodium et concentrée à sec sous pression réduite pour donner 0,78 g de produit brut qui est purifié par filtration sur 20 g de gel de silice en éluant avec un mélange acétate d'éthyle/n-hexane (v/v = 4/6). Après concentration à sec sous pression réduite, on obtient 0,70 g de phényl-4 trichlorométhyl-2 oxazolidine-1,3 carboxylate-5-(4R,5S) d'acétoxy-4 benzoyloxy-2α époxy-5β,20 hydroxy-1 oxo-9 bis-(trichloro-2,2,2 éthoxy) carbonyloxy-7β,10β taxéne-11 yle-13α sous forme d'un mélange de deux diastéréoisomères dont les caractéristiques sont les suivantes:
- spectre infra-rouge (en comprimé avec KBr) : principales bandes d'absorption caractéristiques à 1760, 1730, 1600, 1585, 1490, 1450, 1250, 1065, 980, 810, 760, 725-700 cm⁻¹
- spectre de résonance magnétique nucléaire du proton (400 MHz ; CDCl₃ ; déplacements chimiques δ en ppm ; constantes de couplage J en Hz) (mélange des diastéréoisomères dans les proportions 70/30) 1,15 à 1,30 (mt, 6H) ; 1,84 (s, 1H) ; 1,86 (s, 1H); 2,07 (s, 1H); 2,00 à 2,10 (mt, 1H); 2,15 (s, 1H) ; 2,10 à 2,30 (mt, 2H) ; 2,55 à 2,70 (mt, 1H) ; 3,20 (mf, 1H); 3,32 (mf, 1H) ; 3,87 (d, J = 7, 1H); 3,94 (d, J = 7, 1H); 4,10 (d, J = 8, 1H); 4,13 (d, J = 8, 1H) ; 4,27 (d, J = 8, 1H) ; 4,30 (d, J = 8, 1H); 4,58 (d, J = 7,5, 1H) 4,61 (d, J = 12, 1H); 4,63 (d, J = 12, 1H) ; 4,70 (d, J = 8, 1H); 4,80 (ab, 2H) ; 4,80 (mt, 1H) ; 4,85 à 5,00 (mt, 2H) ; 5,13 (d, J = 7,5, 1H) ; 5,53 (s large, 1H) ; 5,56 (dd, J = 11 et 7, 1H) ; 5,60 (dd, J = 11 et 7, 1H) ; 5,66 (d, J = 7, 1H); 5,68 (d, J = 7, 1H); 6,20 à 6,35 (mt, 1H) ; 6,24 (s, 1H) ; 6,27 (s, 1H) ; 7,30 à 7,50 (mt, 3H) ; 7,30 à 7,70 (mt, 3H) ; 7,60 (d, 2H) ; 8,03 (d, J = 7,5, 2H).

A une solution de 0,50 g de phényl-4 trichlorométhyl-2 oxazolidine-1,3 carboxylate-5-(4S,5R) d'acétoxy-4 benzoyoxy-2α époxy-5β,20 hydroxy-1 oxo-9 bis-(trichloro-2,2,2 éthoxy) carbonyloxy-7β,10β taxène-11 yle-13α dans 5 cm3 d'acétate d'éthyle on ajoute 0,27 g de zinc en poudre et 1,07 cm3 d'acide acétique. La solution est agitée à une température voisine de 20°C pendant 15 heures puis filtrée sur un verre fritté. Le précipité est lavé avec de l'acétate d'éthyle (20 cm3) et la phase organique est successivement lavée à l'eau (15 cm3), avec une solution aqueuse saturée de bicarbonate de sodium (2 fois 15 cm3) puis séchée sur sulfate de sodium. La solution est alors concentrée à sec sous pression réduite à 35°C pour donner 0,33 g d'une solide amorphe. Un dosage par chromatographie liquide haute performance montre que l'amino-3 phényl-3 hydroxy-2 propionate-(2R,3S) d'acétoxy-4 benzoyloxy-2α époxy-5β,20 trihydroxy-1,7β,10β oxo-9 taxène-11 yle-13α titrant 50 %, est obtenu avec un rendement de 65 %.

Les caractéristiques du produit obtenu sont les suivantes :
- spectre de résonance magnétique nucléaire du proton (400 MHz ; DMSO d₆, déplacements chimiques δ en ppm ; constantes de couplage J en Hz) : 0,99 (s, 3H) ; 1,03 (s, 3H) ; 1,53 (s, 3H) ; 1,65 (mt, 1H) ; 1,75 (s, 3H) ; 1,70 à 1,90 (mt, 2H) ; 2,12 (s, 3H) ; 2,28 (mt, 1H) ; 3,65 (d, J = 7, 1H); 4,02 (ab, J = 8, 2H) ; 4,00 à 4,15 (mt, 3H) ; 4,56 (s, 1H) ; 4,90 (d large, J = 10, 1H); 4,99 (s large, 1H) ; 5,05 (mf, 1H) ; 5,10 (s, 1H); 5,42 (d, J = 7, 1H) ; 5,88 (t, J = 9, 1H) ; 7,15 à 7,45 (mt, 5H) ; 7,65 (t, J = 7,5, 2H) ; 7,73 (t, J = 7,5, 1H); 7,98 (d, J = 7,5, 2H).

A une solution de 0,30 g d'amino-3 phényl-3 hydroxy-2 propionate-(2R,3S) d'acétoxy-4 benzoyloxy-2α époxy-5β,20 bihydroxy-1,7β,10β oxo-9 taxène-11 yle-13α brut, obtenu précédemment, dans 5 cm3 de méthanol, on ajoute 0,11 g de diterbutyle dicarbonate. Le mélange réactionnel est agité à une température voisine de 20°C pendant 15 heures, puis on ajoute 20 cm3 d'eau La solution est extraite trois fois avec 15 cm3 de chlorure de méthylène. Les phases organiques réunies sont séchées sur sulfate de sodium puis concentrées à sec sous pression réduite. On obtient ainsi 0,395 g de produit brut. Le dosage par chromatographie liquide haute performance montre que le rendement en tert-butoxycarbonylamino-3 phényl-3 hydroxy-2 propionate-(2R,3S) d'acétoxy-4 benzoyloxy-2α époxy-5β,20 trihydroxy-1,7β,10β oxo-9 taxène-11 yle-13α est de 70 %.

L'acide phényl-4 trichlorométhyl-2 oxazolidine-1,3 carboxylique-5-(4S,5R) peut être préparé de la manière suivante :

Une solution de 3,0 g de tert-butoxycarbonylamino-3 hydroxy-2 phényl-3 propionate-(2R,3S) de méthyle, de 5 cm3 de chloral et de 0,05 g de p-toluènesulfonate de pyridinium dans 40 cm3 de toluène anhydre est chauffée à reflux avec distillation du solvant. On distille 15 cm3 de solvant puis on ajoute 5 cm3 de chloral et 0,05 g de p-toluènesulfonate de pyridinium. On distille 20 cm3 de solvant puis on ajoute 5 cm3 de chloral ainsi que 30 cm3 de toluène anhydre. On distille 25 cm3 de solvant puis on ajoute 5 cm3 de chloral et 35 cm3 de toluène anhydre. On distille 25 cm3 de solvant puis la solution est refroidie à une température voisine de 20°C. La solution organique est lavée à l'eau (2 fois 50 cm3), séchée sur sulfate de sodium et concentrée à sec sous pression réduite à environ 50°C. Le résidu obtenu est purifié par chromatographie liquide sur gel de silice en éluant avec un mélange acétate d'éthyle-cyclohexane (1-3 en volumes). On obtient ainsi, avec un rendement de 91 %, 3,0 g de phényl-4 trichlorométhyl-2 oxazolidine-1,3 méthoxycarbonyl-5-(4S,5R) dont les caractéristiques sont les suivantes :
- spectre infra-rouge (CCl₄) : bandes d'absorption caractéristiques δ 3400, 3100, 3075, 3040, 2960, 1755, 1605, 1590, 1495, 1460, 1440, 1205 et 700 cm⁻¹
- spectre de résonance magnétique nucléaire du proton (200 MHz ; DMSO d₆ ; déplacements chimiques δ en ppm ; constantes de couplage J en Hz) (mélange des diastéréoisomères dans la proportion 65/35) : 3,62 (s, 3H) ; 3,72 (s, 3H) ; 4,50 (d, J = 7,5, 1H) ; 4,50 à 4,70 (mf, 1H); 4,62 (d large, J = 7,5, 1H) ; 4,66 (ab limite, 2H) ; 5,22 (mf, 1H) ; 5,40 (s, 1H); 5,43 (s, 1H) ; 7,30 à 7,70 (mt, 5H).

A une solution de 10,48 g de phényl-4 trichlorométhyl-2 oxazolidine-1,3 méthoxycarbonyl-5-(4S,5R) dans 120 cm3 de méthanol, on ajoute une solution de 1,49 g d'hydroxyde de lithium monohydraté dans 40 cm3 d'eau. La solution est agitée à une température voisine de 20°C pendant 1 heure puis le méthanol est évaporé sous pression réduite à 40°C. La phase aqueuse résiduelle est alors acidifiée avec 35 cm3 d'une solution aqueuse d'acide chlorhydrique 1M. On ajoute alors 80 cm3 d'acétate d'éthyle sous forte agitation. La phase aqueuse est soutirée et extraite de nouveau avec 80 cm3 d'acétate d'éthyle. Les phases organiques sont rassemblées, séchées sur sulfate de sodium et concentrées à sec sous pression réduite. Le résidu obtenu est séché pendant une nuit sous pression réduite à une température voisine de 20°C. On obtient ainsi 10,03 g d'acide phényl-4 trichlorométhyl-2 oxazolidine-1,3 carboxylique-5-(4S,SR) dont les caractéristiques sont les suivantes :
- spectre infra-rouge (CHBr₃) : bandes caractéristiques à 3380, 3325-2240, 1730, 1600, 1495, 1455, 810 et 760 cm⁻¹
- spectre de résonance magnétique nucléaire du proton (200 MHz ; DMSO d₆) ; déplacements chimiques δ en ppm ; constantes de couplage J en Hz) : 4,39 (d, J = 7,5, 1H) ; 4,40 à 4,70 (mt, 2H) ; 5,13 (mt, 1H) ; 5,37 (s, 1H) ; 5,41 (s, H) ; 7,10 à 7,60 (mt, 5H).

## Revendications

1. Procédé de préparation de dérivés du taxane de formule générale : 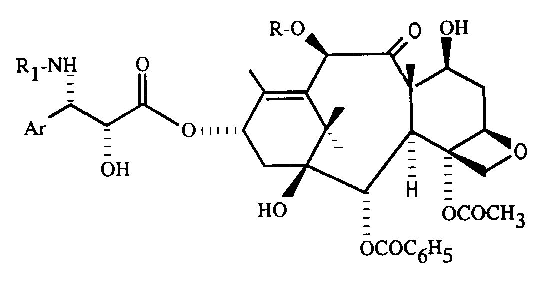 dans laquelle :
R représente un atome d'hydrogène ou un radical acétyle, R₁ représente un radical benzoyle ou un radical R₂-O-CO- dans lequel R₂ représente :
- un radical alcoyle droit ou ramifié contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone ou bicycloalcoyle contenant 7 à 10 atomes de carbone, ces radicaux étant éventuellement substitués par un ou plusieurs substituants choisis parmi les atomes d'halogène et les radicaux hydroxy, alcoyloxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle, cyano, carboxy ou alcoyloxycarbonyle dont la partie alcoyle contient 1 à 4 atomes de carbone,
- ou un radical phényle éventuellement substitué par un ou plusieurs atomes ou radicaux choisis parmi les radicaux alcoyles contenant 1 à 4 atomes de carbone ou alcoyloxy contenant 1 à 4 atomes de carbone,
- ou un radical hétérocyclyle azoté saturé ou non saturé contenant 5 ou 6 chaînons éventuellement substitué par un ou plusieurs radicaux alcoyles contenant 1 à 4 atomes de carbone,
étant entendu que les radicaux cycloalcoyles, cycloalcényles ou bicycloalcoyles peuvent être éventuellement substitués par un ou plusieurs radicaux alcoyles contenant 1 à 4 atomes de carbone, et
Ar représente un radical phényle ou α- ou β-naphtyle éventuellement substitué par un ou plusieurs atomes ou radicaux choisis parmi les atomes d'halogène (fluor, chlore, brome, iode) et les radicaux alcoyles, alcényles, alcynyles, aryles, arylalcoyles, alcoxy, alcoylthio, aryloxy, arylthio, hydroxy, hydroxyalcoyle, mercapto, formyle, acyle, acylamino, aroylamino, alcoxycarbonylamino, amino, alkylamino, dialkylamino, carboxy, alcoxycarbonyle, carbamoyle, dialcoylcarbamoyle, cyano et trifluorométhyle, étant entendu que les radicaux alcoyles et les portions alcoyles des autres radicaux contiennent 1 à 4 atomes de carbone, que les radicaux alcényles et alcynyles contiennent 3 à 8 atomes de carbone et les radicaux aryles sont les radicaux phényles ou *α-* ou β-naphtyles,
caractérisé en ce que :
1) on estérifie un dérivé de la baccatine III ou de la désacétyl-10 baccatine III protégée de formule générale : 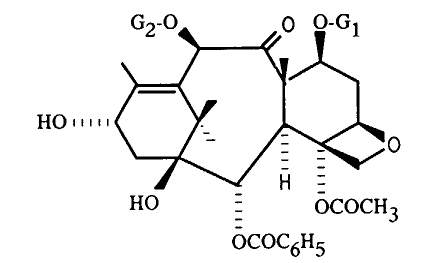 dans laquelle G₁ et éventuellement G₂ représentent un groupement protecteur de la fonction hydroxy, au moyen d'un acide de formule générale : 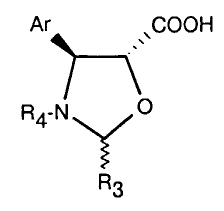 dans laquelle Ar est défini comme précédemment, R₃ représente un radical trihalométhyle ou phényle substitué par un radical trihalométhyle, et R₄ représente un atome d'hydrogène ou est identique à R₁ défini précédemment ou d'un dérivé de cet acide, pour obtenir un produit de formule générale : 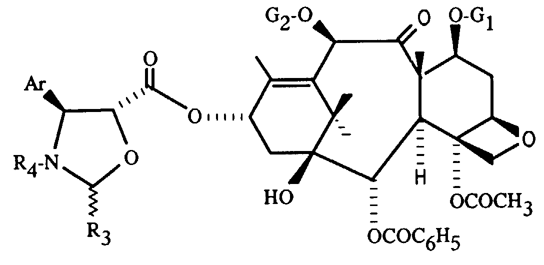 dans laquelle Ar, R₃, R₄, G₁ et G₂ sont définis comme précédemment,
2) on remplace les groupements protecteurs des fonctions hydroxy et amino du produit obtenu par des atomes d'hydrogène, pour obtenir un produit de formule générale : 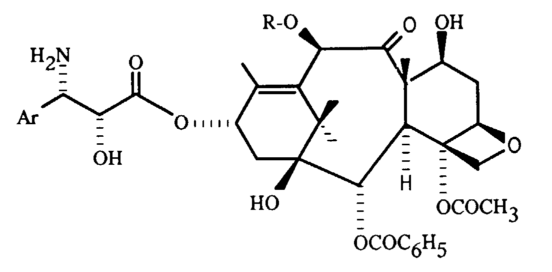 dans laquelle Ar et R sont définis comme précédemment puis
3) traite le produit ainsi obtenu par un réactif permettant d'introduire un substituant R₁ sur la fonction amino, et
4) isole le produit obtenu.

2. Procédé selon la revendication 1 caractérisé en ce que l'estérification est effectuée au moyen d'un acide de formule générale : 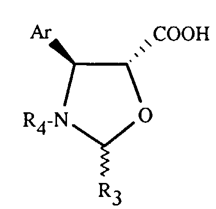 dans laquelle Ar, R₃ et R₄ sont définis comme dans la revendication 1 en opérant en présence d'un agent de condensation et d'un agent d'activation dans un solvant organique à une température comprise entre -10 et 90°C.

3. Procédé selon la revendication 2 caractérisé en ce que l'agent de condensation est choisi parmi les imides et les carbonates réactifs et l'agent d'activation est choisi parmi les aminopyridines.

4. Procédé selon la revendication 3 caractérisé en ce que l'agent de condensation est choisi parmi le dicyclohexylcarbodiimide et le dipyridyl-2 carbonate et l'agent d'activation est choisi parmi la diméthylamino-4 pyridine ou la pyrrolidino-4 pyridine.

5. Procédé selon la revendication 2 caractérisé en ce que le solvant est choisi parmi les éthers, les cétones, les esters, les nitriles, les hydrocarbures aliphatiques, les hydrocarbures aliphatiques halogénés et les hydrocarbures aromatiques.

6. Procédé selon la revendication 5 caractérisé en ce que le solvant est choisi parmi les hydrocarbures aromatiques.

7. Procédé selon la revendication 1 caractérisé en ce que l'estérification est effectuée au moyen d'un anhydride de formule générale : 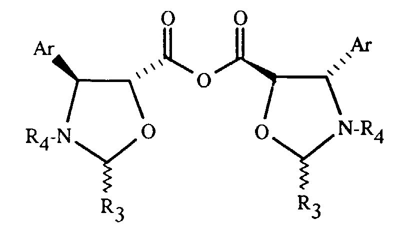 dans laquelle Ar, R₃ et R₄ sont définis comme dans la revendication 1 en opérant en présence d'un agent d'activation dans un solvant organique à une température comprise entre 0 et 90°C.

8. Procédé selon la revendication 7 caractérisé en ce que l'agent d'activation est choisi parmi les aminopyridines.

9. Procédé selon la revendication 8 caractérisé en ce que l'agent d'activation est choisi parmi la diméthylamino4 pyridine ou la pyrrolidino4 pyridine.

10. Procédé selon la revendication 7 caractérisé en ce que le solvant est choisi parmi les éthers, les cétones, les esters, les nitriles, les hydrocarbures aliphatiques, les hydrocarbures aliphatiques halogénés et les hydrocarbures aromatiques.

11. Procédé selon la revendication 1 caractérisé en ce que l'estérification est effectuée au moyen d'un acide activé de formule générale : 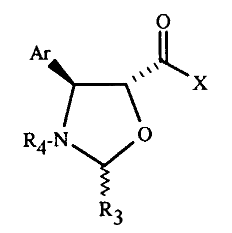 dans laquelle Ar, R₃ et R₄ sont définis comme précédemment et X représente un atome d'halogène ou un radical acyloxy ou aroyloxy, éventuellement préparé in situ, en présence d'une base en opérant dans un solvant organique à une température comprise entre 10 et 80°C.

12. Procédé selon la revendication 11 caractérisé en ce que la base est choisie parmi les bases organiques azotées.

13. Procédé selon la revendication 12 caractérisé en ce que la base organique azotée est choisie parmi les amines tertiaires aliphatiques, la pyridine et les aminopyridines.

14. Procédé selon la revendication 11 caractérisé en ce que le solvant organique est choisi parmi les éthers, les cétones, les esters, les nitriles, les hydrocarbures aliphatiques, les hydrocarbures aliphatiques halogénés et les hydrocarbures aromatiques.

15. Procédé selon la revendication 14 caractérisé en ce que le solvant est choisi parmi les hydrocarbures aromatiques.

16. Procédé selon la revendication 1 caractérisé en ce que le remplacement par des atomes d'hydrogène des groupements protecteurs des fonctions hydroxy et amino est effectué par traitement par le zinc, éventuellement associé à du cuivre, en présence d'acide acétique à une température comprise entre 20 et 60°C.

17. Procédé selon la revendication 1 caractérisé en ce que le remplacement par des atomes d'hydrogène des groupements protecteurs de la fonction hydroxy et amino est effectué au moyen d'un acide minéral ou organique en solution dans un alcool aliphatique contenant 1 à 3 atomes de carbone ou dans un ester aliphatique en présence de zinc éventuellement associé à du cuivre.

18. Procédé selon l'une des revendications 16 ou 17 dans lequel G₁ et éventuellement G₂ représentent un radical trichloro-2,2,2 éthoxycarbonyle ou (trichlorométhyl-2 propoxy)-2 carbonyle.

19. Procédé selon la revendication 1 caractérisé en ce que l'introduction d'un substituant R₁ sur la fonction amino est effectuée par action du chlorure de benzoyle ou d'un dérivé réactif de formule générale :
R₂-O-CO-Y
dans laquelle Y représente un atome d'halogène ou un reste -OR₂ ou -O-CO-R₂ et R₂ est défini comme dans la revendication 1 en opérant dans un solvant organique en présence d'une base minérale ou organique à une température comprise entre 0 et 50°C.

20. Procédé selon la revendication 19 caractérisé en ce que le solvant est choisi parmi les esters aliphatiques et les hydrocarbures aliphatiques halogénés.

21. Procédé selon la revendication 19 caractérisé en ce que la base est le bicarbonate de sodium.

22. Les acides de formule générale : 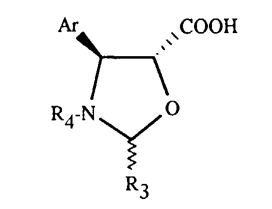 dans laquelle Ar, R₃ et R₄ sont définis comme dans la revendication 1, éventuellement sous forme de sel, d'ester, d'anhydride, d'anhydride mixte ou d'halogénure.

23. Un produit de formule générale 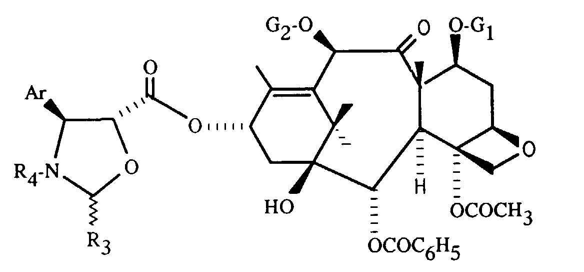 dans laquelle Ar, R₃, R₄, G₁ et G₂ sont définis comme dans la revendication 1.

24. Un produit selon la revendication 23 dans lequel Ar, R₃ et R₄ étant définis comme dans la revendication 1, G₁ représente un radical trichloro-2,2,2 éthoxycarbonyle ou (trichlorométhyl-2 propoxy)-2 carbonyle et G₂ représente un radical acétyle ou un radical trichloro-2,2,2 éthoxycarbonyle ou (trichlorométhyl-2 propoxy)-2 carbonyle.

## Patentansprüche

1. Verfahren zur Herstellung von Taxan-Derivaten der allgemeinen Formel 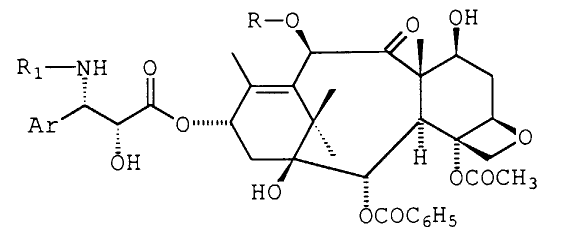 in der
R ein Wasserstoffatom oder einen Acetylrest darstellt, R₁ einen Benzoylrest oder einen Rest R₂-O-CO- bedeutet, worin R₂ ist:
- ein gerader oder verzweigter Rest Alkyl mit 1 bis 8 Kohlenstoffatomen, Alkenyl mit 2 bis 8 Kohlenstoffatomen, Alkinyl mit 3 bis 8 Kohlenstoffatomen, Cycloalkyl mit 3 bis 6 Kohlenstoffatomen, Cycloalkenyl mit 4 bis 6 Kohlenstoffatomen oder Bicycloalkyl mit 7 bis 10 Kohlenstoffatomen, wobei diese Reste gegebenenfalls substituiert sind durch einen oder mehrere Substituenten, ausgewählt unter den Halogenatomen und den Resten Hydroxy, Alkyloxy mit 1 bis 4 Kohlenstoffatomen, Dialkylamino, von dem jeder Alkylteil 1 bis 4 Kohlenstoffatome enthält, Piperidino, Morpholino, 1-Piperazinyl (gegebenenfalls substituiert in -4 durch einen Alkylrest mit 1 bis 4 Kohlenstoffatomen oder durch einen Phenylalkylrest, dessen Alkylteil 1 bis 4 Kohlenstoffatome enthält), Cycloalkyl mit 3 bis 6 Kohlenstoffatomen, Cycloalkenyl mit 4 bis 6 Kohlenstoffatomen, Phenyl, Cyano, Carboxy oder Alkyloxycarbonyl, dessen Alkylteil 1 bis 4 Kohlenstoffatome enthält,
- oder ein Rest Phenyl, gegebenenfalls substituiert durch ein oder mehrere Atome oder Reste, ausgewählt unter den Alkylresten mit 1 bis 4 Kohlenstoffatomen oder den Alkyloxyresten mit 1 bis 4 Kohlenstoffatomen,
- oder ein Rest eines gesättigten oder ungesättigten Stickstoff-Heterocyclus mit 5 oder 6 Ringgliedern, gegebenenfalls substituiert durch einen oder mehrere Alkylreste mit 1 bis 4 Kohlenstoffatomen,
mit der Maßgabe, daß die Reste Cycloalkyl, Cycloalkenyl oder Bicycloalkyl gegebenenfalls durch einen oder mehrere Alkylreste mit 1 bis 4 Kohlenstoffatomen substituiert sein können, und Ar einen Rest Phenyl oder α- oder β-Naphthyl darstellt, gegebenenfalls substituiert durch ein oder mehrere Atome oder Reste, ausgewählt unter den Halogenatomen (Fluor, Chlor, Brom, Iod) und den Resten Alkyl, Alkenyl, Alkinyl, Aryl, Arylalkyl, Alkoxy, Alkylthio, Aryloxy, Arylthio, Hydroxy, Hydroxyalkyl, Mercapto, Formyl, Acyl, Acylamino, Arylamino, Alkoxycarbonylamino, Amino, Alkylamino, Dialkylamino, Carboxy, Alkoxycarbonyl, Carbamoyl, Dialkylcarbamoyl, Cyano und Trifluormethyl, mit der Maßgabe, daß die Alkylreste und Alkylteile der anderen Reste 1 bis 4 Kohlenstoffatome, die Reste Alkenyl und Alkinyl 3 bis 8 Kohlenstoffatome enthalten und die Arylreste Phenylreste oder α- oder β-Naphthylreste sind,
dadurch gekennzeichnet, daß man
1) ein geschütztes Derivat von Baccatin III oder von 10-Desacetyl-Baccatin III der allgemeinen Formel 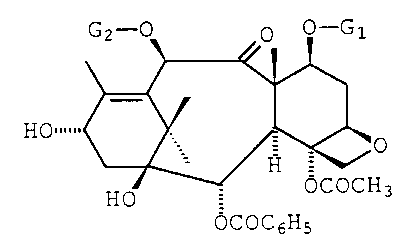 in der G₁ und gegebenenfalls G₂ eine Schutzgruppe für die Hydroxyfunktion darstellt, mit Hilfe einer Säure der allgemeinen Formel 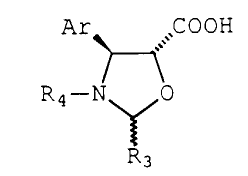 oder einem Derivat dieser Säure verestert, worin Ar wie oben definiert ist, R₃ einen Rest Trihalomethyl oder Phenyl, substituiert durch einen Rest Trihalomethyl, darstellt und R₄ ein Wasserstoffatom bedeutet oder mit R₁, wie oben definiert, identisch ist, um ein Produkt der allgemeinen Formel 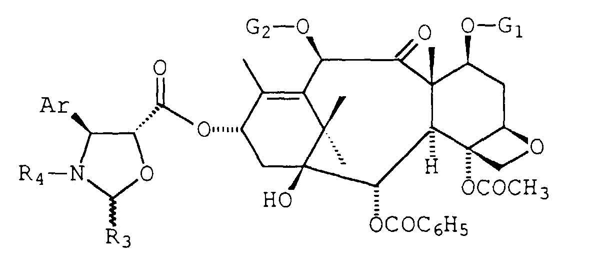 zu erhalten, worin Ar, R₃, R₄, G₁ und G₂ wie oben definiert sind,
2) die Schutzgruppen für die Funktionen Hydroxy und Amino des erhaltenen Produktes durch Wasserstoffatome ersetzt, um ein Produkt der allgemeinen Formel 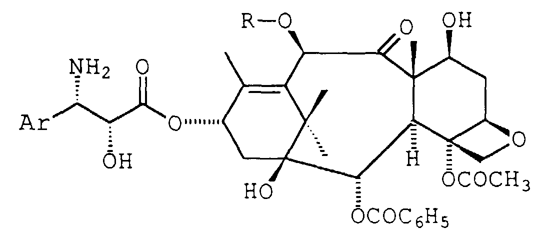 zu erhalten, worin Ar und R wie oben definiert sind, und
3) anschließend das auf diese Weise erhaltene Produkt mit einem Reaktanden behandelt, der ermöglicht, einen Substituenten R₁ bei der Aminofunktion einzuführen, und
4) das erhaltene Produkt isoliert.

2. Verfahren nach Anspruch 1, dadurch gekennzeichnet, daß die Veresterung mit Hilfe einer Säure der allgemeinen Formel 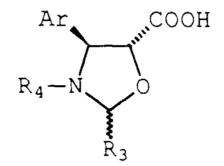 durchgeführt wird, in der Ar, R₃ und R₄ wie in Anspruch 1 definiert sind, wobei man in Anwesenheit eines Kondensationsmittels und eines Aktivierungsmittels in einem organischen Lösungsmittel und bei einer Temperatur zwischen -10 °C und 90 °C arbeitet.

3. Verfahren nach Anspruch 2, dadurch gekennzeichnet, daß das Kondensationsmittel unter den Imiden und den reaktiven Carbonaten und das Aktivierungsmittel unter den Aminopyridinen ausgewählt werden.

4. Verfahren nach Anspruch 3, dadurch gekennzeichnet, daß das Kondensationsmittel unter Dicyclohexylcarbodiimid und 2-Dipyridyl-carbonat und das Aktivierungsmittel unter 4-Dimethylaminopyridin oder 4-Pyrrolidino-pyridin ausgewählt werden.

5. Verfahren nach Anspruch 2, dadurch gekennzeichnet, daß das Lösungsmittel unter den Ethern, den Ketonen, den Estern, den Nitrilen, den aliphatischen Kohlenwasserstoffen, den halogenierten aliphatischen Kohlenwasserstoffen und den aromatischen Kohlenwasserstoffen ausgewählt wird.

6. Verfahren nach Anspruch 5, dadurch gekennzeichnet, daß das Lösungsmittel unter den aromatischen Kohlenwasserstoffen ausgewählt wird.

7. Verfahren nach Anspruch 1, dadurch gekennzeichnet, daß die Veresterung mit Hilfe eines Anhydrides der allgemeinen Formel 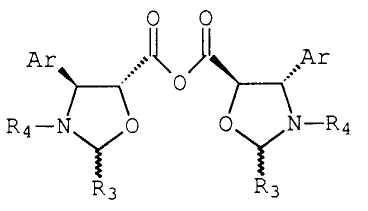 durchgeführt wird, in der Ar, R₃ und R₄ wie in Anspruch 1 definiert sind, wobei man in Anwesenheit eines Aktivierungsmittels in einem organischen Lösungsmittel und bei einer Temperatur zwischen 0 °C und 90 °C arbeitet.

8. Verfahren nach Anspruch 7, dadurch gekennzeichnet, daß das Aktivierungsmittel unter den Aminopyridinen ausgewählt wird.

9. Verfahren nach Anspruch 8, dadurch gekennzeichnet, daß das Aktivierungsmittel unter 4-Dimethylamino-pyridin oder 4-Pyrrolidino-pyridin ausgewählt wird.

10. Verfahren nach Anspruch 7, dadurch gekennzeichnet, daß das Lösungsmittel unter den Ethern, den Ketonen, den Estern, den Nitrilen, den aliphatischen Kohlenwasserstoffen, den halogenierten aliphatischen Kohlenwasserstoffen und den aromatischen Kohlenwasserstoffen ausgewählt wird.

11. Verfahren nach Anspruch 1, dadurch gekennzeichnet, daß die Veresterung mit Hilfe einer aktivierten Säure der allgemeinen Formel 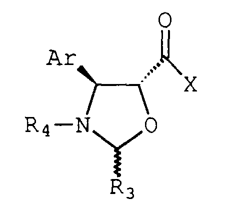 gegebenenfalls hergestellt in situ, durchgeführt wird, in der Ar, R₃ und R₄ wie oben definiert sind und X ein Halogenatom oder einen Rest Acyloxy oder Aryloxy darstellt, wobei man in Anwesenheit einer Base in einem organischen Lösungsmittel und bei einer Temperatur zwischen 10 °C und 80 °C arbeitet.

12. Verfahren nach Anspruch 11, dadurch gekennzeichnet, daß die Base unter den organischen Stickstoffbasen ausgewählt wird.

13. Verfahren nach Anspruch 12, dadurch gekennzeichnet, daß die organische Stickstoffbase unter den tertiären aliphatischen Aminen, Pyridin und den Aminopyridinen ausgewählt wird.

14. Verfahren nach Anspruch 11, dadurch gekennzeichnet, daß das organische Lösungsmittel unter den Ethern, den Ketonen, den Estern, den Nitrilen, den aliphatischen Kohlenwasserstoffen, den halogenierten aliphatischen Kohlenwasserstoffen und den aromatischen Kohlenwasserstoffen ausgewählt wird.

15. Verfahren nach Anspruch 14, dadurch gekennzeichnet, daß das Lösungsmittel unter den aromatischen Kohlenwasserstoffen ausgewählt wird.

16. Verfahren nach Anspruch 1, dadurch gekennzeichnet, daß der Austausch der Schutzgruppen für die Hydroxyfunktion und die Aminofunktion durch Wasserstoffatome mit Hilfe einer Behandlung durch Zink, gegebenenfalls assoziiert mit Kupfer, in Anwesenheit von Essigsäure bei einer Temperatur zwischen 20 °C und 60 °C durchgeführt wird.

17. Verfahren nach Anspruch 1, dadurch gekennzeichnet, daß der Austausch der Schutzgruppen für die Hydroxyfunktion und die Aminofunktion durch Wasserstoffatome mit Hilfe einer Mineralsäure oder organischen Säure in Lösung eines aliphatischen Alkohols mit 1 bis 3 Kohlenstoffatomen oder in einem aliphatischen Ester in Anwesenheit von Zink, gegebenenfalls assoziiert mit Kupfer, durchgeführt wird.

18. Verfahren nach einem der Ansprüche 16 oder 17, worin G₁ und gegebenenfalls G₂ einen Rest 2,2,2-Trichlor-ethoxycarbonyl oder 2-(2-Trichlormethyl-propoxy)-carbonyl darstellen.

19. Verfahren nach Anspruch 1, dadurch gekennzeichnet, daß die Einführung eines Substituenten R₁ bei der Aminofunktion durch Umsetzung mit Benzoylchlorid oder einem reaktiven Derivat der allgemeinen Formel
R₂-O-CO-Y
durchgeführt wird, in der Y ein Halogenatom oder einen Rest -OR₂ oder -O-CO-R₂ darstellt und R₂ wie in Anspruch 1 definiert ist, wobei man in einem organischen Lösungsmittel in Anwesenheit einer Mineralbase oder organischen Base und bei einer Temperatur zwischen 0 °C und 50 °C arbeitet.

20. Verfahren nach Anspruch 19, dadurch gekennzeichnet, daß das organische Lösungsmittel unter den aliphatischen Estern und den halogenierten aliphatischen Kohlenwasserstoffen ausgewählt wird.

21. Verfahren nach Anspruch 19, dadurch gekennzeichnet, daß die Base Natriumbicarbonat ist.

22. Die Säuren der allgemeinen Formel 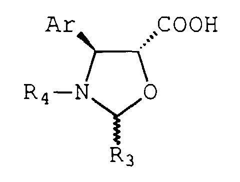 worin Ar, R₃ und R₄ wie in Anspruch 1 definiert sind, gegebenenfalls in Form von Salz, Ester, Anhydrid, gemischtem Anhydrid oder Halogenid.

23. Ein Produkt der allgemeinen Formel 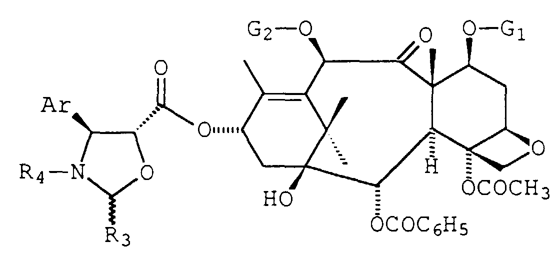 in der Ar, R₃, R₄, G₁ und G₂ wie in Anspruch 1 definiert sind.

24. Ein Produkt nach Anspruch 23, worin Ar, R₃ und R₄ wie in Anspruch 1 definiert sind, G₁ einen Rest 2,2,2-Trichlor-ethoxycarbonyl oder 2-(2-Trichlormethyl-propoxy)-carbonyl darstellt und G₂ einen Acetylrest oder einen Rest 2,2,2-Trichlor-ethoxycarbonyl oder 2-(2-Trichlormethyl-propoxy)-carbonyl bedeutet.

## Claims

1. Process for the preparation of taxane derivatives of general formula: 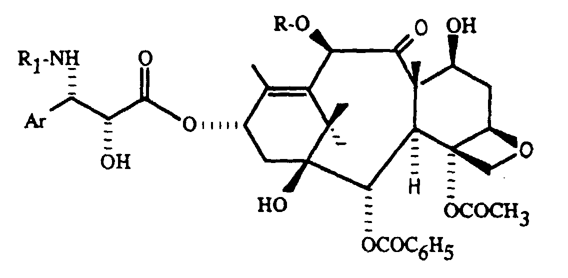 in which:
R represents a hydrogen atom or an acetyl radical, R₁ represents a benzoyl radical or a radical R₂-O-CO- in which R₂ represents:
- a straight or branched alkyl radical containing 1 to 8 carbon atoms, an alkenyl radical containing 2 to 8 carbon atoms, an alkynyl radical containing 3 to 8 carbon atoms, a cycloalkyl radical containing 3 to 6 carbon atoms, a cycloalkenyl radical containing 4 to 6 carbon atoms or a bicycloalkyl radical containing 7 to 10 carbon atoms, these radicals optionally being substituted by one or a number of substituents chosen from the halogen atoms and the hydroxyl radical, alkyloxy radical containing 1 to 4 carbon atoms, dialkylamino radical, each alkyl part of which contains 1 to 4 carbon atoms, piperidino radical, morpholino radical, 1-piperazinyl radical (optionally substituted in the 4-position by an alkyl radical containing 1 to 4 carbon atoms or by a phenylalkyl radical, the alkyl part of which contains 1 to 4 carbon atoms), cycloalkyl radical containing 3 to 6 carbon atoms, cycloalkenyl radical containing 4 to 6 carbon atoms, phenyl radical, cyano radical, carboxyl radical or alkyloxycarbonyl radical, the alkyl part of which contains 1 to 4 carbon atoms,
- or a phenyl radical optionally substituted by one or a number of atoms or radicals chosen from the alkyl radicals containing 1 to 4 carbon atoms or the alkyloxy radicals containing 1 to 4 carbon atoms,
- or a saturated or unsaturated nitrogen-containing heterocyclyl radical containing 5 or 6 members, optionally substituted by one or a number of alkyl radicals containing 1 to 4 carbon atoms,
it being understood that the cycloalkyl, cycloalkenyl or bicycloalkyl radicals may optionally be substituted by one or a number of alkyl radicals containing 1 to 4 carbon atoms, and
Ar represents a phenyl or α- or β-naphthyl radical optionally substituted by one or a number of atoms or radicals chosen from the halogen atoms (fluorine, chlorine, bromine or iodine) and the alkyl, alkenyl, alkynyl, aryl, arylalkyl, alkoxy, alkylthio, aryloxy, arylthio, hydroxyl, hydroxyalkyl, mercapto, formyl, acyl, acylamino, aroylamino, alkoxycarbonylamino, amino, alkylamino, dialkylamino, carboxyl, alkoxycarbonyl, carbamoyl, dialkylcarbamoyl, cyano and trifluoromethyl radicals, it being understood that the alkyl radicals and the alkyl portions of the other radicals contain 1 to 4 carbon atoms, that the alkenyl and alkynyl radicals contain 3 to 8 carbon atoms and the aryl radicals are phenyl or α- or β-naphthyl radicals,
characterized in that:
1) a derivative of the protected baccatin III or of the protected 10-deacetylbaccatin III of general formula: 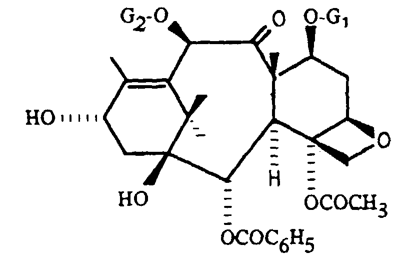 in which G₁ and optionally G₂ represent a protective group of the hydroxyl functional group, is esterified using an acid of general formula: 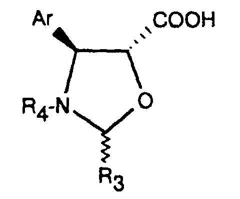 in which Ar is defined as above, R₃ represents a trihalomethyl radical or phenyl radical substituted by a trihalomethyl radical, and R₄ represents a hydrogen atom or is identical to R₁ defined above, or a derivative of this acid, to produce a product of general formula: 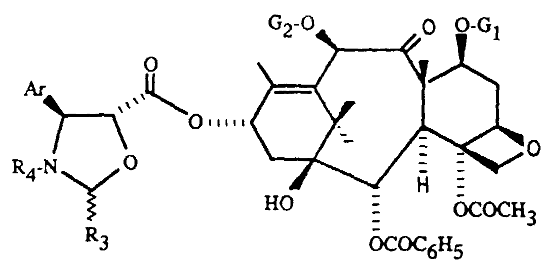 in which Ar, R₃, R₄, G₁ and G₂ are defined as above,
2) the protective groups of the hydroxyl and amino functional groups of the product obtained are replaced by hydrogen atoms to produce a product of general formula: 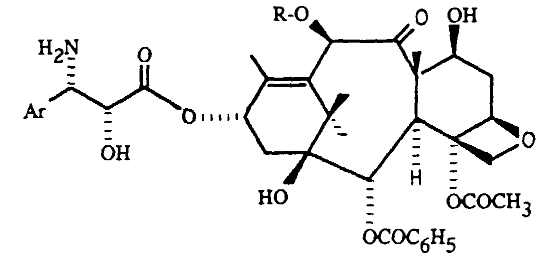 in which Ar and R are defined as above, then
3) the product thus obtained is treated with a reagent which makes it possible to introduce a substituent R₁ onto the amino functional group, and
4) the product obtained is isolated.

2. Process according to claim 1, characterized in that the esterification is carried out using an acid of general formula: 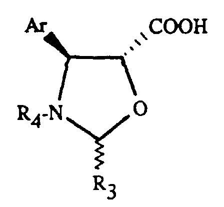 in which Ar, R₃ and R₄ are defined as in claim 1, the reaction being carried out in the presence of a condensation agent and of an activating agent in an organic solvent at a temperature between -10 and 90°C.

3. Process according to claim 2, characterized in that the condensation agent is chosen from imides and reactive carbonates and the activating agent is chosen from aminopyridines.

4. Process according to claim 3, characterized in that the condensation agent is chosen from dicyclohexylcarbodiimide and di-2-pyridyl ketone and the activating agent is chosen from 4-dimethylaminopyridine or 4-pyrrolidinopyridine.

5. Process according to claim 2, characterized in that the solvent is chosen from ethers, ketones, esters, nitriles, aliphatic hydrocarbons, halogenated aliphatic hydrocarbons and aromatic hydrocarbons.

6. Process according to claim 5, characterized in that the solvent is chosen from aromatic hydrocarbons.

7. Process according to claim 1, characterized in that the esterification is carried out using an anhydride of general formula: 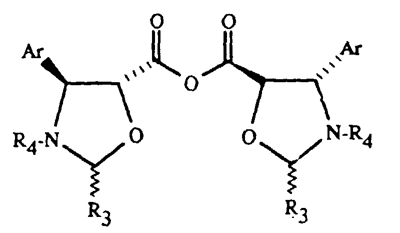 in which Ar, R₃ and R₄ are defined as in claim 1, the reaction being carried out in the presence of an activating agent in an organic solvent at a temperature between 0 and 90°C.

8. Process according to claim 7, characterized in that the activating agent is chosen from aminopyridines.

9. Process according to claim 8, characterized in that the activating agent is chosen from 4-dimethylaminopyridine or 4-pyrrolidinopyridine.

10. Process according to claim 7, characterized in that the solvent is chosen from ethers, ketones, esters, nitriles, aliphatic hydrocarbons, halogenated aliphatic hydrocarbons and aromatic hydrocarbons.

11. Process according to claim 1, characterized in that the esterification is carried out using an activated acid of general formula: 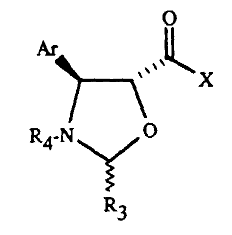 in which Ar, R₃ and R₄ are defined as above and X represents a halogen atom or an acyloxy or aroyloxy radical, optionally prepared in situ, in the presence of a base, the reaction being carried out in an organic solvent at a temperature between 10 and 80°C.

12. Process according to claim 11, characterized in that the base is chosen from nitrogenous organic bases.

13. Process according to claim 12, characterized in that the nitrogenous organic base is chosen from aliphatic tertiary amines, pyridine and aminopyridines.

14. Process according to claim 11, characterized in that the organic solvent is chosen from ethers, ketones, esters, nitriles, aliphatic hydrocarbons, halogenated aliphatic hydrocarbons and aromatic hydrocarbons.

15. Process according to claim 14, characterized in that the solvent is chosen from aromatic hydrocarbons.

16. Process according to claim 1, characterized in that replacement by hydrogen atoms of the protective groups of the hydroxyl and amino functional groups is carried out by treatment with zinc, optionally in combination with copper, in the presence of acetic acid at a temperature between 20 and 60°C.

17. Process according to claim 1, characterized in that replacement by hydrogen atoms of the protective groups of the hydroxyl and amino functional group is carried out using an inorganic or organic acid in solution in an aliphatic alcohol containing 1 to 3 carbon atoms or in an aliphatic ester in the presence of zinc, optionally in combination with copper.

18. Process according to either of claims 16 or 17, in which G₁ and optionally G₂ represent a 2,2,2-trichloroethoxycarbonyl or 2- (2- (trichloromethyl)propoxy)-carbonyl radical.

19. Process according to claim 1, characterized in that the introduction of a substituent R₁ onto the amino functional group is carried out by reacting with benzoyl chloride or with a reactive derivative of general formula:
R₂-O-CO-Y
in which Y represents a halogen atom or a residue -OR₂ or -O-CO-R₂ and R₂ is defined as in claim 1, the reaction being carried out in an organic solvent in the presence of an inorganic or organic base at a temperature between 0 and 50°C.

20. Process according to claim 19, characterized in that the solvent is chosen from aliphatic esters and halogenated aliphatic hydrocarbons.

21. Process according to claim 19, characterized in that the base is sodium bicarbonate.

22. The acids of general formula: 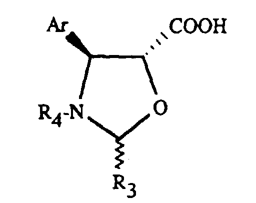 in which Ar, R₃ and R₄ are defined as in claim 1, optionally in the salt, ester, anhydride, mixed anhydride or halide form.

23. A product of general formula 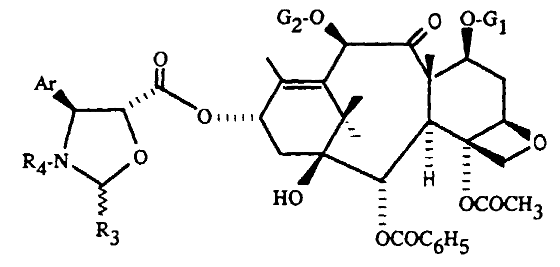 in which Ar, R₃, R₄, G₁ and G₂ are defined as in claim 1.

24. A product according to claim 23, in which, Ar, R₃ and R₄ being defined as in claim 1, G₁ represents a 2,2,2-trichloroethoxycarbonyl or 2-(2-(trichloromethyl)-propoxy)carbonyl radical and G₂ represents an acetyl radical or a 2,2,2-trichloroethoxycarbonyl or 2-(2-(trichloromethyl)propoxy)carbonyl radical.
